(19) Europäisches Patentamt
European Patent Office
Office européen des brevets

(11) Publication number: **0 036 331 B2**

(12) **NEW EUROPEAN PATENT SPECIFICATION**

(45) Date of publication of the new patent specification: **18.10.89**

(51) Int. Cl.⁴: **C 07 D 295/02**

(21) Application number: **81301126.9**

(22) Date of filing: **17.03.81**

(54) **Synthesis of morpholine and derivatives thereof via the reaction of dialkylene glycol and ammonia.**

(30) Priority: **17.03.80 US 130782**

(43) Date of publication of application: **23.09.81 Bulletin 81/38**

(45) Publication of the grant of the patent: **01.08.84 Bulletin 84/31**

(45) Mention of the opposition decision: **18.10.89 Bulletin 89/42**

(84) Designated Contracting States: **AT BE CH DE FR GB IT LI LU NL SE**

(56) References cited:
DE-A- 2 738 115
DE-A- 2 758 769
DE-C- 1 941 633
DE-C- 2 040 507
SU-A- 175 512
US-A- 3 151 112
US-A- 3 154 544

CHEMICAL ABSTRACTS, vol. 75, no. 23, December 6, 1971, oage 327, abstract 140871h, COLUMBUS, OHIO (US)

(73) Proprietor: **AIR PRODUCTS AND CHEMICALS, INC., P.O. Box 538, Allentown, Pennsylvania 18105 (US)**

(72) Inventor: **Daughenbaugh, Randall Jay, Box 334A, RD No. 1 Barton, PA 19505 (US)**
Inventor: **Dixon, Dale David, Box 53D RD No. 2, Kutztown, PA 19530 (US)**
Inventor: **Fowlkes, Robert Lee, 220 Cedar Street, Milton Florida 32570 (US)**

(74) Representative: **Burford, Anthony Frederick et al, W.H. Beck, Greener & Co. 7 Stone Buildings Lincoln's Inn, London WC2A 3SZ (GB)**

**EP 0 036 331 B2**

## Description

Technical field

This invention relates to an improved process for forming morpholine and alkyl and/or phenyl substituted derivatives thereof.

Description of the prior art

U.S. Patent 2 412 209 discloses a process for producing aliphatic amines from alcohols and particularly morpholine by the reaction of diethylene glycol and ammonia. Temperatures from 160-400 °C are used and the reaction is carried out in the presence of a hydrogenation catalyst. Examples of hydrogenation catalysts suited for the reaction include Raney nickel, copper chromite, copper-nickel-chromite, iron, cobalt, etc. Liquid or gas phase conditions are suggested.

U.S. Patent 3 154 544 discloses the preparation of substituted morpholines by the vapor phase conversion of a dialkylene glycol having at least one secondary hydroxyl group with hydrogen, and ammonia, in the presence of a hydrogenation/dehydrogenation catalyst. It is noted in the reference that diethylene glycol could not be converted to morpholine by reaction with ammonia in substantial conversion or yield, particularly under conditions suggested in the prior art e.g. U.S. 2 412 209 or 2 529 923.

U.S. Patent 3 155 657 discloses a process for producing polyglycolamines and morpholine by the reaction of diethylene glycol and ammonia. Temperatures of 150-350°C, pressures of $20 \times 10^5 - 51 \times 10^6$ Pa (20-500 atmospheres) and a contact time of from 5 minutes to 4 hours are suggested with pressures of $69 \times 10^5 - 23 \times 10^6$ Pa (1000-3000 psig) being used. The reaction was carried out preferably in the presence of a ruthenium catalyst. Yields of morpholine ranged from about 14-77% with glycol conversions of from about 48-96%.

U.S. Patent 3 151 112 shows a process for producing morpholine and derivatives by the reaction of dialkylene glycols, e.g. diethylene glycol with ammonia at temperatures of 150-400 °C, and pressures of $30 \times 10^5$-$41 \times 10^6$ Pa (30-400 atmospheres) while maintaining liquid phase conditions. Ammonia is added in large excess to that of stoichiometric requirements. Yields of up to about 50% morpholine at the high reaction pressures are shown.

U.S. Patent 3 151 113 discloses a process of preparing N-alkyl morpholine products by the reaction of hydroxy or amino terminated diethylene ether feed-stocks with ammonia in the presence of a hydrogenation catalyst under liquid phase conditions. Pressures of $34 \times 10^5$-$34 \times 10^6$ Pa (500-5000 psig) and temperatures of 150-300 °C are employed. Conventional hydrogenation/dehydrogenation catalysts are used and these may be supported on alumina, kieselguhr, and other various supports or unsupported.

Japanese Patent Publication No. 46-32 188, discloses a process for producing morpholine by the reaction of diethylene glycol and ammonia. In carrying out the process the reactants are charged to an autoclave and reacted at 240 °C and $25 \times 10^5$ Pa (25 atmospheres) in the presence of hydrogen. The improved process relates to the use of a Raney-nickel catalyst having sufficient aluminium therein to consume by-product water as it is produced. The effect of water removal is to extend the catalyst life of the Raney-nickel.

U.S. Patent 4 091 218 discloses a process for recovering ammonia from a gas stream resulting from the catalytic reaction of ammonia and a dialkylene glycol as described in U.S. 3 151 112. In the process the recovery of the product is effected by contacting the reaction effluent gas stream containing unreacted hydrogen, ammonia, and methane with a dialkylene glycol feed stock under conditions for adsorbing ammonia and leaving anhydrous hydrogen and methane.

British Patent 1 530 570 discloses a process for producing (2-aminoalkoxy)-alkanol (AEE) and morpholine derivatives from ammonia and oxydialkanol under pressures sufficient to maintain liquid conditions. Temperature and pressure are controlled in order to vary the quantity of the aminoethoxyethanol and morpholine derivative produced. Temperatures generally are 200-220 °C while gauge pressures of at least $48 \times 10^5$ Pa (700 psig) are used. Ammonia to alkanol ratios of 6:1 are used, with the ammonia being in the anhydrous form. Hydrogen is added to maintain catalyst activity.

Summary of the invention

This invention relates to an improved process for forming heterocyclic amines selected from morpholine and its alkyl and /or phenyl substituted derivatives. The basic process comprises reacting a dialkylene glycol and ammonia in the presence of hydrogen and a hydrogenation/dehydrogenation catalyst at conventional temperatures. The improvement constituting the basis of the invention resides in continuously passing the reactants to a trickle-bed catalytic reactor, operating the reactor so that the reaction conditions do not exceed by more than 10 °C the dew point of the feed whereby at least 1% of the dialkylene glycol is maintained as discontinuous liquid phase (i.e. a liquid phase which is not continuous but consists of discrete quantities, usually drops, of liquid and at least a portion of the heterocyclic amine is present in the vapour phase) and continuously removing product. Preferably the conditions are maintained such that the heterocyclic amine is predominantly in the gas phase.

According to a first aspect of the present invention, there is provided a process for producing morpholine or an alkyl of phenyl substituted derivative thereof by contacting an alcohol of the formula

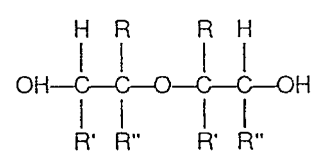

where R, R', and R″ may be identical or different, each representing a hydrogen atom, alkyl or phenyl radicals, with ammonia in a reactor said reaction being carried out in the presence of hydrogen and a hydrogenation-dehydrogenation catalyst, characterised in that:

(a) the dialkylene glycol, ammonia and hydrogen are continuously passed downflow through a trickle bed reactor;

(b) the reactor is operated so that the reaction conditions do not exceed by more than 10°C the dew point of the feed whereby at least 1% of the dialkylene glycol is maintained as a discontinuous liquid phase and at least a portion of the morpholine is present in the vapour phase; and

(c) heterocyclic amine product is continuously removed from the reactor.

According to a second aspect of the present invention, there is provided a process for preparing morpholine by contacting an alcohol which is diethylene glycol and/or 2-(2-amino-ethoxy) ethanol with ammonia in a reactor in the presence of hydrogen and a hydrogenation-dehydrogenation catalyst, characterised in that:

(a) the alcohol, ammonia and hydrogen are continuously passed downflow trough a trickle bed reactor;

(b) the reactor is operated under conditions so that the reaction conditions do not exceed by more than 10°C the dew point of the feed whereby at least 1% of the alcohol is maintained as a discontinuous liquid phase and at least a portion of the morpholine is present in the vapour phase; and

(c) morpholine is continuously removed from the reactor.

Several advantages are associated with the improved process of this invention as compared to the prior art. These include:

the reaction permits substantially complete conversion of the dialkylene glycol, particularly diethylene glycol in the manufacture of morpholine, thereby minimizing recovery problems and minimizing recycle;

the reaction conditions are moderate e.g. low pressures are used thereby resulting in an energy saving as compared to prior art processes operating under high pressure, liquid phase conditions; and

high selectivity to the heterocyclic amine i.e. morpholine and its derivatives, with little formation of heavies in the form of polyamines, e.g. morpholino diethylene glycol (MDEG) and bismorpholino diethylene glycol (BMDEG) is achieved.

Brief description of the drawings

Figure 1 is plot of the product distribution in gas chromatograph (GC) area percent obtained by the reaction of diethylene glycol and ammonia versus temperature.

Figure 2 is a plot of product distribution in gas chromatograph (GC) area percent versus ammonia-diethylene glycol-hydrogen mole concentration in the feed.

Description of the preferred embodiments

The feed component suited for practicing the process is a dialkylene glycol of the formula

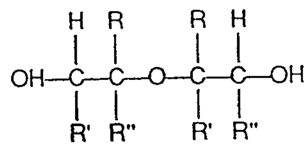

where R, R', and R″ may be identical or different. each representing a hydrogen atom, alkyl or phenyl radicals. R, R', R″ contain typically from 1 to 6 carbon atoms, if alkyl, and preferably not more than 2 carbon atoms. For purposes of producing a commercially important heterocyclic amine, i.e. morpholine, the dialkylene glycol is diethylene glycol (DEG). Others, result in the production of alkyl and phenyl substituted morpholine derivatives. Specific examples of preferred dialkylene glycols include diethylene glycol, dipropylene glycol and dibutylene glycol.

As with other processes, the reaction of dialkylene glycol to form heterocyclic amines is carried out in the presence of ammonia. Ammonia to dialkylene glycol ratios, on a molar basis, are at least 1:1 and up to 100:1, but preferably 4 to 16:1. While the process requires at least equal molar amounts of ammonia to glycol to permit reaction on a stoichiometric basis, molar ratios higher than 16 to 20:1 do not result in significant advantages. Because of the unique nature of the reaction conditions for carrying out the process, higher ratios of ammonia to glycol can have a detrimental effect in commercial units in that such higher ratios require increased pressures.

The presence of hydrogen is necessary for the proper and efficient conduct of the process. It is used in combination with ammonia and it is believed its function is to maintain catalyst activity. Molar ratios of ammonia to hydrogen generally are from 4 to 60:1 and preferably 6 to 32:1. Low ratios of ammonia to hydrogen, e.g. 2:1 to 4:1 generally result in increased heavies formation. It is believed lower ammonia to hydrogen ratios reduce the ammonia content in the liquid phase thereby permitting any residual liquid phase morpholine to react and form heavies. Such is also true with the introduction of other inert gases such as nitrogen or methane. They, like hydrogen, reduce the ammonia content in the liquid phase.

The catalysts suited for practicing the invention include those commonly used in prior art processes provided that they are wettable with the dialkylene glycol under the reaction conditions. By wettable, it is meant the catalyst will permit the formation of a very thin, liquid film about the surface of the catalyst as required in a trickle bed. The hydrogenation/dehydrogenation catalysts suited for practicing the process generally includes one or more metals from copper, nickel, cobalt, chromium, molybdenum, manganese, platinum, palladium, ruthenium, and rhodium. The preferred catalysts i.e. those which are most effective for the reactant are nickel, cobalt, and copper or contain such components.

Most of the above hydrogenation/dehydrogenation metals, even in highly porous form, will not permit the formation of thin film of dialkylene glycol about its surface, but rather will cause it to bead up on the surface. In those cases, the metal should be impregnated or incorporated into a wettable support. The support for the hydrogenation-dehydrogenation catalyst then is (a) one which is inert to the extent that it is not soluble or reactable with the reaction medium and (b) one which is wettable by the dialkylene glycol. Supports suited include silica, alumina, kieselguhr, and others conventionally used in the art. Alumina and silica are preferred. Broadly, the proportion of hydrogenation/dehydrogenation metal by weight of the catalyst, including support, is from 0.01% to 70% and typically between 20 to 40%. This level may vary to the extent the catalyst loses its wettability.

In practicing the process, the temperature and pressure are maintained in the catalytic reaction zone such that some, at least 1% preferably at least 5%, of the reactant dialkylene glycol is in the liquid phase, while the heterocyclic product is predominantly in the vapor phase, e.g. greater than 80 mole% and preferably 90% assuming 90% conversion of the dialkylene glycol and 75% of the intermediate if one is formed. In addition, the temperature and pressure are selected so the reaction conditions do not substantially exceed (greater than about 10 °C) the dew point temperature of the feed.

In a preferred mode for carrying out the process, i.e., that of maintaining some of the dialkylene glycol in the liquid phase with the predominant portion of the heterocyclic amine in the vapor phase, the reactants are fed downflow through a trickle-bed reactor such that the dialkylene glycol is present as a discontinuous liquid phase. This inhibits flooding of the bed and hold up of gaseous product. Using this technique, the conversion of dialkylene glycol to a morpholine derivative is high and the percentage of heavies in the form of polyamines (MDEG and BMDEG) is low.

While not intending to be bound by theory, it is believed the presence of the 2 phase system between the dialkylene glycol reactant and the product amine in the trickle bed permits high conversion of dialkylene glycol and high selectivity with little formation of heavies. By maintaining some of the dialkylene glycol and ammonia, to some extent, in the liquid phase and because of the dialkylene glycols inherent attraction via wetting of the catalyst surface the actual contact time of the dialkylene glycol in the reaction zone is extended. On the other hand, as the dialkylene glycol is converted to heterocyclic amine and because the heterocyclic amine is rapidly removed from the reaction zone to the vapor phase it, in effect, has a short contact time. Therefore, since the heterocyclic amine concentration in the liquid phase is relatively low vis-à-vis the dialkylene glycol, and the residence time of the heterocyclic amine in the liquid phase is short, the amine is effectively precluded from reacting with the glycol to form heavies. In addition, the reaction rate is enhanced because the reaction conditions also provide for expulsion of water in addition to the morpholine via the gas phase thus shifting the equilibrium to product.

When one operates under higher pressure, e.g. greater than $34 \times 10^5$ Pa (500 psig), morpholine becomes part of the liquid phase, at least in a proportion greater than 40% at conventional operating temperatures, and becomes available for reaction with the DEG. Thus, in the prior art the only way to reduce the amount of heavies was to increase the pressure substantially and bring the ammonia into the liquid phase. The high mole ratio of ammonia, plus its presence in the liquid phase, in effect reduces the possibility of the product morpholine to react with DEG and form heavies.

The optimum liquid-vapor phase properties of the product are to be calculated assuming 90% dialkylene glycol conversion and 71% intermediate conversion for the major components present in the process (e.g. present as a feed or product mixture). These values were selected since these are representative of the actual process conditions. However, as the feed conditions approach the dew point, the 90% conversion assumption tends to detract from the importance of the liquid/vapor equilibrium data. To gain a better idea of the actual conditions, it may be necessary to select a lower value, e.g. 50%.

In the case for producing morpholine, these major components are diethylene glycol, ammonia, hydrogen, 2-(2-aminoethoxy) ethanol (AEE intermediate) and by-products, e.g. morpholino diethylene glycol (MDEG) and bis-morpholino diethylene glycol (BMDEG). The liquid-vapor phase properties for the feed are based upon the feed components themselves. The vapor-liquid equilibrium for feed and products is determined from the equation:

Vapor-liquid equilibria

$$Ø_I Y_I P = {}_I X_I F_I^o e^{\frac{(P\bar{V}_I)}{RT}}$$

$Ø_I$ = Vapor fugacity coefficient

I = Liquid activity coefficient

$F_I^o$ = Standard state liquid fugacity

$\bar{V}_I$ = Liquid partial molar volume

e = Natural base

$$K_I = \frac{Y_I}{X_I} = \frac{I^{F^\circ} I\, e^{\frac{(P\bar{V}_I)}{RT}}}{Ø_I P}$$

The fugacity coefficient is solved by the use of the Virial equation of state and its application can be found in an article by Zellner et al, appearing in "Industrial Engineering Chemical Fundamentals", volume 9, November 9, 549-564 (1970).

The standard state liquid fugacity is defined in the following equation:

Standard state liquid fugacity

$$F_I^o = \frac{\emptyset_{IS}\,Ps}{e\left(\frac{PsV}{RT}\right)}$$

wherein Ø, is the pure component vapor phase fugacity coefficient, Ps is the pure component vapor pressure, e is natural base. This expression can be used where actual vapor pressure data are known. Otherwise a generalized correlation of the form below is used.

Generalized correlation

$$\frac{F^o}{P_c} = F(T_R, W)$$

$P_c$ = Critical pressure
$T_R$ = Reduced temperature
W = Acentric factor

The Generalized Correlation and its use is discussed in the Zellner et al. article.

The UNIQUAC Model equation is used to calculate the activity coefficient. The equation model for a binary system is:

Uniquac equation

$$LN_I = LN\frac{\emptyset_I}{X_I} + \frac{Z}{2}Q_I\,LN\frac{\Theta_I}{\emptyset_I} + \emptyset_J\left(L_I - \frac{R_I}{R_J}LJ\right)$$

$$-Q_I\,LN\,(O_I + O_J T_{JI}) + \Theta_J\,Q_I\left(\frac{TJI}{\Theta_I + \Theta_J T_{JI}} - \frac{TIJ}{\Theta_J + \Theta_I T_{IJ}}\right)$$

$R_I$ = Volume parameter of molecule I
$Q_I$ = Area parameter of molecule I
$\emptyset_I$ = Volume fraction of molecule I; $\emptyset_J$ refers to molecule J
$\Theta_I$ = Area fraction of molecule I
Z = Coordination number, set equal to 10
T = Adjustable binary parameter obtained from regression of vapor liquid equilibrium data
= Liquid activity coefficient
LN = Natural logarithm
$L_I = \frac{Z}{2}(R_I - Q_I) - R_I - 1$

Activity coefficients for a multicomponent mixture can be determined from an extension of the equation model for a binary system using only binary adjustable parameters. No ternary (or higher) constants are required with this model. When vapor-liquid equilibrium data for individual binaries are not available, the adjustable binary parameters $T_{IJ}$'s are set equal to unity.

A description of the application of the UNIQUAC Model is found in Abrams et al., AICHE Journal, Volume 21, Number 116-128 (1975).

The reactions to product morpholine and byproducts are believed to be as follows and provide a good reference for the discussion to follow regarding the reaction.

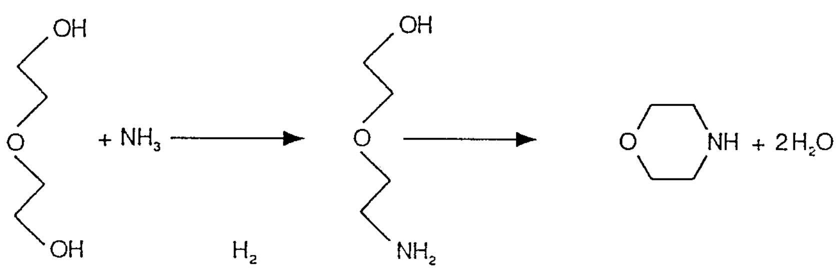


A.

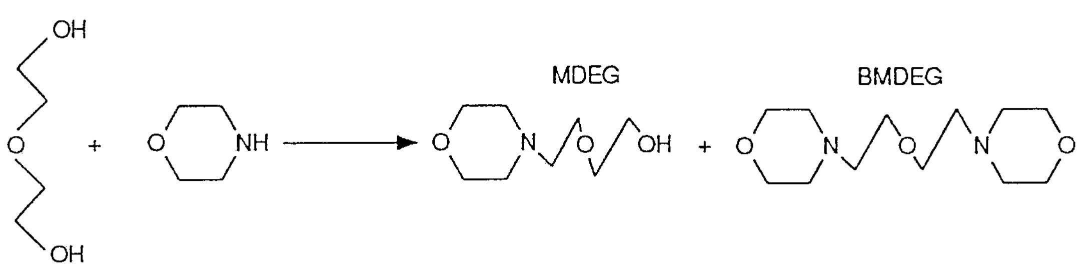


B.

From the reaction formulas A and B, it can be postulated that selectivity to the heterocyclic amine is largely dependent upon the concentration of ammonia in the liquid phase and in contact with the dialkylene glycol at the catalyst site or absence of heterocyclic amine. The increased concentration of ammonia shifts the reaction equilibrium to 2-(2-aminoethoxy) ethanol (intermediate) which then converts to morpholine. The removal of the heterocyclic amine from the liquid phase also enhances selectively because the heterocyclic amine is not available for reaction with the dialkylene glycol or possibly aminoethoxyethanol or MDEG.

It is also postulated from the reaction formulas that the process conditions facilitate a shift in reaction equilibrium toward the product in view of the fact that the heterocyclic amine, as well as the water, is removed via vaporization from the liquid reaction zone. Water inherently is vaporized under the specified conditions and it too is removed from the liquid reaction zone. In the past, this shift in reaction equilibrium was achieved by incorporating aluminum or other dehydrating component in the catalyst system. (NOTE: Japanese Patent Publication 46-32 188). The removal of the product heterocyclic amine from the reaction zone also contributes to this increase in conversion to the product side.

To permit the maintenance of an appropriate contact time in the reaction zone for the conversion of dialkylene glycol to the heterocyclic amine, the reaction is generally carried out at a liquid hourly space velocity of from 0.05 to 2.5 $hr^{-1}$. (Liquid hourly space velocity (LHSV) is defined as the ratio of the volume of liquid dialkylene glycol per volume of catalyst per hour.) The liquid hourly space velocity is not as critical as some other parameters in the process in that it is largely dependent upon the activity of the catalyst. In those instances where the catalyst is highly reactive, a higher liquid hourly space velocity can be utilized to achieve greater throughput. Alternatively, where a catalyst having lower activity is used, lower space velocities are employed. Generally, liquid hourly space velocity is adjusted to permit the greatest conversion based on desired throughput. Commercially, it is possible to operate at a lower conversion and obtain greater product yield in view of the increased throughput through the reactor. Of course this will result in increasing the amount of by-product material coming from the reactor that must be recycled or recovered. A preferred LHSV range for cobalt or nickel containing catalysts is from 0.2 to 1.0 $hr^{-1}$.

The pressure used for the reaction is adjusted to meet desired vapor-liquid criteria for the reactants and products. In addition, the pressure must be adjusted to provide for a desired rate of reaction. Pressures generally suited for commercial operation are from $86 \times 10^4$–$34 \times 10^5$ Pa (125 to 500 psig). However, pressures generally higher than $21 \times 10^5$ Pa (300 psig) are not used as they show no significant improvement in the tricke bed reactor. Pressures above $34 \times 10^5$ Pa (500 psig) can result in increased heavies formation. Preferred pressures are $14 \times 10^5$–$21 \times 10^5$ Pa (200–300 psig).

The temperature used for carrying out the reaction generally is from 140 to 280°C at the pressure specified. Of course as the pressure is increased, temperature can be increased to the extent the vapor-liquid equilibrium criteria is met. Typically the temperature used is from 200 to 250°C. Higher temperatures often cause coking of the catalyst or deactivation.

The following Examples are representative of the preferred embodiments of the invention.

Example 1

Runs of different feedstocks and under different conditions were made in a process design unit reactor which consisted of 1.04 cm (0.41 inch) (I.D.) 304 stainless steel tubing encased in aluminium block. The reactor was Model R-100 designed by the Ace Catalyst Company. The reactor utilized electrical heat for temperature control.

In the runs, diethylene glycol, ammonia and hydrogen were passed over a catalyst containing 42% nickel oxide carried on a gamma alumina support. The catalyst was crushed and sized to 1.68–1.00 mm (12–18 mesh). The nickel oxide was reduced at 399°C (750°F) with hydrogen. The surface area was approximately 190 $m^2$ per gram and supplied under the trademark HSC-102B by the Houdry Division of Air Products and Chemicals Inc. The reactor was charged with 10 cc catalyst to provide a reactor zone bed depth of about 10 centimeters.

In the process the reactor was operated at a variable LHSV based on diethylene glycol as well as variable hydrogen to diethylene glycol to ammonia molar feed ratios. Hydrogen feed rates were measured in ml/min at STP whereas ammonia and DEG were measured in ml/hr. Product distribution is given in gas chromatograph area percent.

The feed DEG, including $NH_3$ and $H_2$ was passed downflow through the reactor at various temperatures ranging from 190–260°C. The liquid DEG remained in the discontinuous phase except in Run 23 which is a comparative run at 10°C above the dew point. Conversion results are shown in Table I and the vapour-liquid data for some of the runs are shown in Table 2. DEG represents diethylene glycol, MOR represents morpholine and AEE represents 2-(2-aminoethoxy) ethanol. The liquid-vapor equilibrium values were calculated assuming 90% diethylene glycol and 71% AEE conversion.

With respect to Table 2, K represents the value for a component as previously described in the formula. The values for DEG and $NH_3$ are the moles liquid for these components at the reaction conditions. L/F refers to the total moles liquid at feed conditions divided by total moles feed and is expressed in percent; $NH_3$/F refers to the ratio of ammonia in the liquid phase divided by

the total moles feed. The values MOR and AEE vapors are the moles of each component in the vapor phase. Total moles product refers to the moles of DEG, MOR, AEE, water and ammonia that would be present assuming 90% conversion of DEG and 71% conversion of AEE. The value % MOR refers to the percent of morpholine in the vapor phase. $NH_3$/MOR refers to the ratio of the moles ammonia in the liquid product to the moles liquid morpholine in the Product. MOR refers to moles morpholine product in the liquid phase.

Table 1

| Run | Temp. °C | $10^5$ Pa Press | Psig Press | ml/min STP $H_2$ | ml/hr DEG | ml/hr $NH_3$ | $H_2$/DEG/$NH_3$ Mole Ratio | (DEG) LHSV | Concentration GC area % MOR | AEE | DEG | MDEG | Others |
|---|---|---|---|---|---|---|---|---|---|---|---|---|---|
| 1 | 240 | 17 | 250 | 15 | 7.5 | 18 | 0.5/1/8 | 0.5 | 32 | 31 | 29 | 3.5 | 4.4 |
| 2 | 250 | 17 | 250 | 15 | 7.5 | 18 | 0.5/1/8 | 0.5 | 37 | 23 | 30 | 1.3 | 8.3 |
| 3 | 260 | 17 | 250 | 15 | 7.5 | 18 | 0.5/1/8 | 0.5 | 25 | 23 | 41 | 1.3 | 10 |
| 4 | 190 | 19 | 280 | 44 | 3.8 | 6.8 | 3/1/6 | 0.25 | 31 | 21 | 30 | 14 | 3.7 |
| 5 | 195 | 19 | 280 | 44 | 3.8 | 6.8 | 3/1/6 | 0.25 | 28 | 24 | 36 | 10.4 | 1.2 |
| 6 | 200 | 14 | 200 | 44 | 3.8 | 6.8 | 3/1/6 | 0.25 | 21 | 25 | 42 | 8.6 | 3.1 |
| 7 | 205 | 19 | 280 | 44 | 3.8 | 6.8 | 3/1/6 | 0.25 | 21 | 20 | 36 | 15 | 3.2 |
| 8 | 210 | 10 | 140 | 44 | 3.8 | 6.8 | 3/1/6 | 0.25 | 26 | 19 | 35 | 15 | 4.2 |
| 9 | 210 | 24 | 350 | 44 | 3.8 | 6.8 | 3/1/6 | 0.25 | 31 | 20 | 29 | 19 | 1.4 |
| 10 | 210 | 19 | 280 | 44 | 3.8 | 6.8 | 3/1/6 | 0.25 | 29 | 19 | 31 | 17 | 4.1 |
| 11 | 210 | 19 | 280 | 15 | 3.8 | 9.2 | 1/1/8 | 0.25 | 29 | 30 | 32 | 7.2 | 1.4 |
| 12 | 210 | 19 | 280 | 30 | 3.8 | 8.1 | 2/1/7 | 0.25 | 27 | 25 | 35 | 12 | 1.7 |
| 13 | 210 | 19 | 280 | 7.5 | 3.8 | 9.8 | 0.5/1/8.5 | 0.25 | 26.2 | 32 | 37 | 3.7 | 1.2 |
| 14 | 210 | 19 | 280 | 7.5 | 3.8 | 18.4 | 0.5/1/16 | 0.25 | 20 | 38 | 38 | 1.5 | 2.5 |
| 15 | 210 | 19 | 280 | 105 | 3.8 | 2.3 | 7/1/2 | 0.25 | 17 | 5.4 | 40 | 36* | 1.5 |
| 16 | 210 | 19 | 280 | 44 | 3.8 | 6.8 | 3/1/6 | 0.25 | 22 | 22 | 41 | 11 | 3.9 |
| 17 | 220 | 19 | 280 | 7.5 | 3.8 | 9.8 | 0.5/1/8.5 | 0.25 | 78 | 7.6 | - | 9.8** | 5.0 |
| 18 | 200 | 17 | 250 | 20 | 7.1 | 28 | 0.72/1/13 | 0.47 | 0.6 | 11 | 86 | 0.6 | 1.5 |
| 19 | 220 | 17 | 250 | 20 | 7.1 | 28 | 0.72/1/13 | 0.47 | 13 | 31 | 51 | 2.5 | 3.1 |
| 20 | 230 | 17 | 250 | 20 | 7.1 | 28 | 0.72/1/13 | 0.47 | 28 | 31 | 33 | 4.9* | 4.3 |
| 21 | 240 | 17 | 250 | 20 | 7.1 | 28 | 0.72/1/13 | 0.47 | 47 | 21 | 17 | 6.5* | 9.2 |
| 22 | 250 | 17 | 250 | 20 | 7.1 | 28 | 0.72/1/13 | 0.47 | 63 | 8.8 | 7.7 | 7.1* | 13 |
| 23 | 260 | 17 | 250 | 20 | 7.1 | 28 | 0.72/1/13 | 0.47 | 2.3 | 16 | 76 | 0.1 | 5.7 |
| 24 | 220 | 17 | 250 | 20 | 7.1 | 28 | 0.72/1/13 | 0.47 | 5.8 | - | - | Catalyst Coked | |
| 25 | 220 | 58 | 850 | 12 | 6.3 | 15 | 0.5/1/8 | 0.25 | 54 | 22 | 13 | 8.5 | 3.1 |
| 26 | 220 | 38 | 550 | 12 | 6.3 | 15 | 0.5/1/8 | 0.25 | 52 | 22 | 15 | 6.1 | 4.5 |
| 27 | 220 | 19 | 270 | 12 | 6.3 | 15 | 0.5/1/8 | 0.25 | 56 | 22 | 15 | 4.8 | 2.8 |
| British Patent 1,530,570 | | | | | | | | | | | | Heavies | |
| 28 | 209 | 48 | 700 | - | - | - | 0.9/1/6.4[v] | 0.61 | 49 | 18 | 15 | 16 | 2.3 |
| 29 | 210 | 97 | 1400 | - | - | - | 0.9/1/6.34[v] | 0.59 | 45 | 20 | 26 | 7.0 | 2.1 |
| 30 | 210 | 181 | 2625 | - | - | - | 0.9/1/5.97[v] | 0.61 | 28 | 17 | 50 | 3.5 | 1.9 |

[v] Reactor feed also includes 6.6 moles $H_2O$/mole DEG and 0.3 moles $N_2$/mole DEG.
* includes some BMDEG

Table 2

| Run | °C Dew Point | K value $NH_3$ | Feed Moles Liq. DEG | $NH_3$ | L/F% | $NH_3$/F% |
|---|---|---|---|---|---|---|
| 1 | 266 | 7.01 | 0.56 | 0.084 | 6.9 | 0.8 |
| 4 | 273 | 5.84 | 0.93 | 0.154 | 12.2 | 1.54 |
| 8 | 244 | 10.6 | 0.6 | 0.04 | 7.94 | 0.48 |
| 9 | 283 | 5.03 | 0.89 | 0.16 | 12.3 | 1.6 |
| 10 | 273 | 5.98 | 0.86 | 0.12 | 11.2 | 1.24 |
| 11 | 269 | 5.54 | 0.85 | 0.16 | 10.6 | 1.6 |
| 12 | 270 | 5.75 | 0.85 | 0.14 | 10.9 | 1.45 |
| 13 | 268 | 5.4 | 0.84 | 0.17 | 10.4 | 1.78 |

Table 2

| | °C | K value | Feed Moles Liq. | | | |
|---|---|---|---|---|---|---|
| Run | Dew Point | $NH_3$ | DEG | $NH_3$ | L/F% | $NH_3$/F% |
| 14 | 249 | 5.3 | 0.71 | 0.15 | 5.6 | 0.88 |
| 15 | 281 | 7.1 | 0.89 | 0.04 | 13.0 | 0.4 |
| 16 | 273 | 5.98 | 0.86 | 0.12 | 11.2 | 1.2 |
| 20 | 250 | 6.6 | 0.48 | 0.07 | 3.8 | 0.53 |
| 21 | 250 | 7.0 | 0.28 | 0.04 | 2.2 | 0.27 |
| 22 | 250 | 7.3 | 0.017 | 0.002 | 0.13 | 0.016 |
| 23 | 250 | 7.3 | 0.0 | 0 | 0 | 0 |
| 25 | 321 | 2.3 | 0.94 | 0.66 | 17.6 | 6.9 |
| 26 | 299 | 3.2 | 0.9 | 0.37 | 14.0 | 3.9 |
| 27 | 268 | 5.9 | 0.79 | 0.14 | 10.1 | 1.4 |
| 28 | 319 | 2.7 | 0.96 | 0.52 | 19.5 | 6.3 |
| 29 | 378 | 1.7 | 0.98 | 1.3 | 31.1 | 15.0 |
| 30 | – | 1.24 | 0.998 | 4.01 | 67.8 | 48.4 |

Table 2 (continued)

| | | | | | Vapor | | | | |
|---|---|---|---|---|---|---|---|---|---|
| Run | Total Moles/ Product | Moles Total Liq. | MOR | $NH_3$/MOR | MOR | AEE | % MOR | K valve MOR | K valve $NH_3$ |
| 1 | 9.93 | 0 | – | – | 0.63 | 0.26 | 100 | | |
| 4 | 10.63 | 0.566 | 0.10 | 0.51 | 0.53 | 0.18 | 83.8 | 0.29 | 5.38 |
| 8 | 10.63 | 10.63 | 0.63 | – | – | – | 100 | 0.64 | 10.1 |
| 9 | 10.63 | 0.43 | 0.07 | 0.57 | 0.56 | 0.14 | 88.8 | 0.34 | 5.05 |
| 10 | 10.63 | 0.27 | 0.038 | 0.57 | 0.6 | 0.16 | 94 | 0.41 | 6.05 |
| 11 | 10.63 | 0.23 | 0.031 | 0.86 | 0.6 | 0.17 | 94.9 | 0.43 | 5.66 |
| 12 | 10.63 | 0.25 | 0.034 | 0.73 | 0.6 | 0.17 | 94.5 | 0.42 | 5.85 |
| 13 | 10.63 | 0.22 | 0.029 | 0.95 | 0.6 | 0.17 | 95.3 | 0.43 | 5.57 |
| 14 | 18.1 | – | – | – | 0.63 | 0.26 | 100 | 0.40 | 5.3 |
| 15 | 10.63 | 1.12 | 0.05 | 0.1 | 0.58 | 0.13 | 91.9 | 0.38 | 7.05 |
| 16 | 10.63 | 0.27 | 0.038 | 0.57 | 0.6 | 0.16 | 93.8 | 0.41 | 6.05 |
| 20 | 15.3 | 0 | – | – | 0.63 | 0.26 | 100 | 0.44 | 5.98 |
| 21 | 15.3 | 0 | – | – | 0.63 | 0.26 | 100 | 0.44 | 5.98 |
| 22 | 15.3 | 0 | – | – | 0.63 | 0.26 | 100 | 0.44 | 5.98 |
| 23 | 15.3 | 0 | – | – | 0.63 | 0.26 | 100 | 0.44 | 5.98 |
| 25 | 9.93 | 1.23 | 0.22 | 1.65 | 0.41 | 0.04 | 64 | 0.25 | 2.4 |
| 26 | 9.93 | 0.62 | 0.08 | 1.2 | 0.52 | 0.09 | 81.3 | 0.31 | 3.38 |
| 27 | 9.93 | 0.07 | 0.009 | 0.95 | 0.629 | 0.17 | 98.4 | 0.52 | 6.0 |
| 28 | 8.88 | 1.33 | 0.27 | 0.84 | 0.36 | 0.028 | 56.3 | 0.49 | 3.56 |
| 29 | 8.88 | 3.57 | 0.50 | 3 | 0.50 | 0.255 | 20.6 | 0.17 | 1.68 |
| 30 | 8.88 | 8.88 | 0.639 | 8.5 | – | – | 0 | 0.59 | 1.9 |

In analyzing the results in Table 1, it should be noted that conversion of diethylene glycol to morpholine increases with increasing temperature e.g. Runs 18–22 except where the temperature is increased to the point where substantially all of the reactants are in the gas phase, e.g. Run 23. At that point, i.e. when the reactants are above the dew point, (about 10°C) the conversion of DEG to product morpholine tends to fall off dramatically.

Figure 1 is a plot which shows the feature.

Table 2 shows the moles liquid DEG ranges from about 1.7% (Run 22) to a maximum of about 90% (Run 4). Typically the range is about 1–60%.

Runs 1, 3, 12, 15–17 and particularly Runs 11–16 show the importance of keeping the ratio of ammonia to DEG high. All of these runs can be combined to show that as the molar ratio of ammonia to DEG high. All of these runs can be combined to show that as the molar ratio of ammonia to DEG is reduced below 4:1, the proportion of heavies increases with the highest proportion being noted in Run 15. From the data ammonia to DEG molar ratios of 4–16:1 provide reasonably good results.

The importance of ammonia ultimately in the liquid phase (generally this is a function of ammonia mole ratio), shows up in the $NH_3$/MOR ra-

tio of Table 2. Selectively generally decreases as the $NH_3$/MOR ratio falls as noted with Run 15.

Runs 25, 26 and 27 as a group show the importance of keeping the pressure low to avoid the formation of heavy by-products. Yield loss to MDEG nearly doubles when pressures are increased from $19 \times 10^5$–$59 \times 10^5$ Pa (270–850 psig). Runs 8, 9 and 10 show the same trend but the effect is much less at the lower $97 \times 10^5$–$24 \times 10^5$ Pa (140–350 psig) level. This point is also confirmed from Runs 29, 30 and 31 taken from Example 1 of British Patent 1 530 570. (The results reported were recalculated making some assumptions to place them on the same basis reported in Table 1).

They show that at pressures of about $48 \times 10^5$ Pa (700 psig) a large amount of heavies are formed. Higher pressures are required to suppress heavies formation since calculations show that at about $48 \times 10^5$ Pa (700 psig) and a mole ratio of about 1/1/6 the morpholine content in the liquid phase in the percent is highest. Higher pressures are required to reduce the percentage of morpholine in the liquid phase.

It should be noted that the numbers given for moles liquid and vapor etc. are not precise as they often are based upon assumptions for the adjustable binary parameters in the equations. However, the numbers do show trends as evidenced by the differences noted a low pressures and those used in the British patent. Greater accuracy is noted in the % MOR values as in those instances a small change in the moles morpholine in the liquid phase does not drastically affect the overall numerical value. As a result the data in Table 2 shows that were the % MOR is high e.g. greater than 90% (assuming about plus or minus 5%) accuracy the selectivity is much higher than where % MOR is lower e.g. 83%. Compare Runs 1 and 4.

Example 2

The procedure of Example 1 was repeated except that a calcium promoted HSC 102B catalyst was used, the calcium being added by washing the HSC 102B catalyst with aqueous calcium nitrate and then drying the catalyst. The conditions utilized in the reactions were: temperature 210 °C, pressure $20 \times 10^5$ Pa (295 psig), and an LHSV based on the diethylene glycol of 0.25 $hour^{-1}$.

The ratio of $NH_3 + H_2$/DEG was kept constant at 9 (except for Run 14 where it was 16.5) while the ratio of $NH_3$ plus $H_2$ was varied. Figure 2 is a plot of some of the results in gas chromatograph area percent for selected components vs the mole ratio of ammonia to hydrogen as mole percent. Also provided are feed molar ratios of ammonia, diethylene glycol and hydrogen.

Figure 2 shows, as did Example 1, that at low ammonia to feed diethylene glycol mole ratios, conversion to morpholine is relatively low. Morpholine conversion (line 1) begins to increase when the molar ratio of ammonia increases to a value of from about 6 to 9 moles per mole diethylene glycol.

MDEG and BMDEG by-product is represented by line 2, and if one were to extrapolate line 2 to include all heavies, essentially a straight line relationship would be noted as shown in line 3. This data also shows that heavies concentration can be reduced for a given reaction condition by increasing the ammonia concentration.

Example 3

The procedure of Example 1 was repeated except that a temperature of 220 °C, a pressure of about $18 \times 10^5$ Pa (250 psia), and a feed ratio of 8:1:0.5 of ammonia:DEG:$H_2$ was utilized. The variable was residence time and that was varied from an LHSV of 1 to a low of 0.25 $hours^{-1}$.

The results are as follows:

Effect of Residence Time on Morpholine Yield and Production Rate
Catalyst = HSC-102B
Mole Ratio $NH_3$:DEG:$H_2$ = 8:1:0.5

| DEG LHSV | MOR Selectivity | MOR Single Pass Yield | Production Rates, g/cc/hr MOR | DGA | DEG | X's |
|---|---|---|---|---|---|---|
| 0.5 | 91 | 69 | 0.31 | 0.11 | 0.005 | 0.047 |
| 0.25 | 87 | 77 | 0.17 | 0.024 | 0.003 | 0.037 |
| 1.0 | 97 | 32 | 0.29 | 0.53 | 0.185 | 0.031 |
| 0.5 | 93 | 56 | 0.25 | 0.14 | 0.059 | 0.035 |

The above results show that selectivity to morpholine decreases with decreasing space velocity. Conversion on the other hand increases with decreasing space velocity. The data also shows that good single space yields are possible with this proces (X's represents by-product).

**Claims**

1. A process for producing morpholine or an alkyl or phenyl substituted derivative thereof by contacting an alcohol of the formula

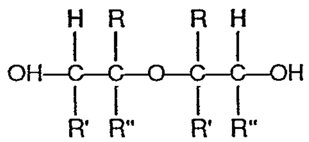

where R, R', and R" may be identical or different, each representing a hydrogen atom, alkyl or phenyl radicals, with ammonia in a reactor said reaction being carried out in the presence of hydrogen and a hydrogenation-dehydrogenation catalyst, characterised in that:

(a) the dialkylene glycol, ammonia and hydrogen are continuously passed downflow through a trickle bed reactor;

(b) the reactor is operated so that the reaction conditions do not exceed by more than 10°C the dew point of the feed whereby at least 1% of the dialkylene glycol is maintained as a discontinuous liquid phase and at least a portion of the heterocyclic amine is present in the vapour phase; and

(c) heterocyclic amine product is continuously removed from the reactor.

2. A process for preparing morpholine by contacting an alcohol which is diethylene glycol and/or 2-(2-amino-ethoxy) ethanol with ammonia in a reactor in the presence of hydrogen and a hydrogenation-dehydrogenation catalyst, characterised in that;

(a) the alcohol, ammonia and hydrogen are continuously passed downflow through a trickle bed reactor;

(b) the reactor is operated under conditions so that the reaction conditions do not exceed by more than 10°C the dew point of the feed whereby at least 1% of the alcohol is maintained as a discontinuous liquid phase and at least a portion of the morpholine is present in the vapour phase; and

(c) morpholine is continuously removed from the reactor.

3. A process as claimed in Claim 1, characterised in that the reactor is operated at a temperature of 140° to 280°C and a pressure of $8.6 \times 10^5$ to $34 \times 10^5$ Pa (125 to 500 psig).

4. A process as claimed in Claim 2, characterised in that the reactor is operated at a temperature of 140° to 280°C and a pressure of $8.6 \times 10^5$ to $34 \times 10^5$ Pa (125 to 500 psig).

5. A process as claimed in any one of the preceding Claims, characterised in that more than 80 mole percent of the heterocyclic amine formed during the reaction is in the vapor phase.

6. A process as claimed in any one of the preceding Claims, characterised in that the alcohol is diethylene glycol.

7. A process as claimed in any one of Claims 1, 2, 5 and 6, characterised in that the temperature maintained in the reaction zone is from 140 to 280°C.

8. A process as claimed in Claim 7, characterised in that the pressure maintained in the reaction zone is from $8.6 \times 10^5$–$34 \times 10^5$ Pa (125 to 500 psig).

9. A process as claimed in any one of the preceding Claims, characterised in that the liquid hourly space velocity, based on alcohol feed, is from 0.05 to 2.5.

10. A process as claimed in any one of the preceding Claims, characterised in that the hydrogenation-dehydrogenation catalyst is wettable by the alcohol and is carried on a support selected from alumina, silica and mixtures thereof.

11. A process as claimed in Claim 10, characterised in that said hydrogenation-dehydrogenation catalyst carried upon the support contains a component selected from nickel, cobalt and chromium.

**Patentansprüche**

1. Verfahren zur Herstellung von Morpholin oder eines alkyl- oder phenylsubstituierten Derivats davon durch Kontaktieren eines Alkohols der Formel

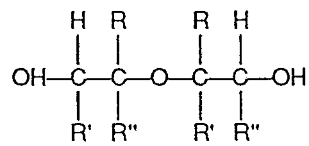

worin R, R', R", die identisch oder verschieden sein können, jeweils ein Wasserstoffatom, Alkyl- oder Phenylreste repräsentieren, mit Ammoniak in einem Reaktor, wobei die Reaktion in Gegenwart von Wasserstoff und eines Hydrierungs/Dehydrierungs-Katalysators durchgeführt wird, dadurch gekennzeichnet, dass

a) das Dialkylenglykol, Ammoniak und Wasserstoff kontinuierlich stromabwärts durch einen Rieselbett-Reaktor geführt werden,

b) der Reaktor so betrieben wird, dass die Reaktionsbedingungen den Taupunkt der Beschikkung um nicht mehr als 10°C übersteigen, wobei mindestens 1% des Dialkylenglykols in Form einer diskontinuierlichen, flüssigen Phase aufrechterhalten werden, und mindestens ein Teil des heterocyclischen Amins in der Dampfphase vorhanden ist und

c) das heterocyclische Amin-Produkt kontinuierlich aus dem Reaktor entfernt wird.

2. Verfahren zur Herstellung von Morpholin durch Kontaktieren eines Alkohols, bei dem es sich um Diethylenglykol und/oder 2-(2-Aminoethoxy)ethanol handelt, mit Ammoniak in einem Reaktor in Gegenwart von Wasserstoff und eines Hydrierungs-Dehydrierungs-Katalysators, dadurch gekennzeichnet, dass

a) der Alkohol, Ammoniak und Wasserstoff kontinuierlich stromabwärts durch einen Rieselbett-Reaktor geführt werden,

b) der Reaktor unter solchen Bedingungen betrieben wird, dass die Reaktionsbedingungen den Taupunkt der Beschickung um nicht mehr als 10°C übersteigen, wobei mindestens 1% des Alkohols in Form einer diskontinuierlichen, flüssigen Phase aufrechterhalten werden und zumindest ein Teil des Morpholins in der Dampfphase

vorhanden ist und

c) das Morpholin kontinuierlich aus dem Reaktor entfernt wird.

3. Verfahren nach Anspruch 1, dadurch gekennzeichnet, dass der Reaktor bei einer Temperatur von 140 bis 280°C und einem Druck von $8{,}6 \times 10^5$ bis $34 \times 10^5$ Pa (125 bis 500 psig) betrieben wird.

4. Verfahren nach Anspruch 2, dadurch gekennzeichnet, dass der Reaktor bei einer Temperatur von 140 bis 280°C und einem Druck von $8{,}6 \times 10^5$ bis $34 \times 10^5$ Pa (125 bis 500 psig) betrieben wird.

5. Verfahren nach einem der vorhergehenden Ansprüche, dadurch gekennzeichnet, dass mehr als 80 Mol-% des während der Reaktion gebildeten heterocyclischen Amins in der Gasphase (Dampfphase) vorliegen.

6. Verfahren nach einem der vorhergehenden Ansprüche, dadurch gekennzeichnet, dass es sich bei dem Alkohol um Diethylenglykol handelt.

7. Verfahren nach einem der Ansprüche 1, 2, 5 und 6, dadurch gekennzeichnet, dass die in der Reaktionszone aufrechterhaltene Temperatur 140 bis 280°C beträgt.

8. Verfahren nach Anspruch 7, dadurch gekennzeichnet, dass der in der Reaktionszone aufrechterhaltene Druck $8{,}6 \times 10^5$ bis $34 \times 10^5$ Pa (125 bis 500 psig) beträgt.

9. Verfahren nach einem der vorhergehenden Ansprüche, dadurch gekennzeichnet, dass die stündliche Flüssigkeitsraumgeschwindigkeit, bezogen auf die Alkoholbeschickung, 0,05 bis 2,5 beträgt.

10. Verfahren nach einem der vorhergehenden Ansprüche, dadurch gekennzeichnet, dass der Hydrierungs-Dehydrierungs-Katalysator durch den Alkohol benetzbar ist und auf einen Träger, ausgewählt aus Aluminiumoxid, Siliciumdioxid und Mischungen davon, aufgebracht ist.

11. Verfahren nach Anspruch 10, dadurch gekennzeichnet, dass der auf den Träger aufgebrachte Hydrierungs-Dehydrierungs-Katalysator eine Komponente, ausgewählt aus Nickel, Kobalt und Chrom, enthält.

12. Verfahren nach Anspruch 11, dadurch gekennzeichnet, dass es sich bei dem Hydrierungs-Dehydrierungs-Katalysator um einen auf Aluminiumoxid als Träger aufgebrachten Nickelkatalysator handelt.

13. Verfahren nach einem der vorhergehenden Ansprüche, dadurch gekennzeichnet, dass die Reaktionszone bei 200 bis 250°C gehalten wird und dass der Druck $14 \times 10^5$ bis $21 \times 10^5$ Pa (200 bis 300 psig) beträgt.

14. Verfahren nach einem der vorhergehenden Ansprüche, dadurch gekennzeichnet, dass es sich bei dem heterocyclischen Amin um Morpholin handelt und dass mindestens 90% der Gesamtmole an Morpholin in dem Produkt in der Gasphase (Dampfphase) vorliegen.

15. Verfahren nach einem der vorhergehenden Ansprüche, dadurch gekennzeichnet, dass das Molverhältnis von Ammoniak zu Alkohol in den Reaktoren 4:1 bis 16:1 beträgt.

**Revendications**

1. Procédé de production de morpholine ou d'un dérivé substitué par un radical alcoyle ou phényle de celle-ci, par contact d'un alcool représenté par la formule:

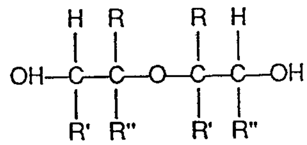

dans laquelle R, R′ et R″ peuvent être identiques ou différents, représentant chacun un atome d'hydrogène, un groupe alcoyle ou phényle, avec de l'ammoniac dans un réacteur, cette réaction étant réalisée en présence d'hydrogène et d'un catalyseur d'hydrogénation-déshydrogénation, caractérisé en ce que:

(a) on fait passer en continu le dialcoyleneglycol, l'ammoniac et l'hydrogène à travers un réacteur à lit à ruissellement;

(b) on fait fonctionner le réacteur de telle sorte que les conditions de réaction ne dépassent pas de plus de 10°C le point de rosée de l'alimentation moyennant quoi au moins 1% du dialcoylèneglycol est maintenu sous forme de phase liquide discontinue et au moins une portion de l'amine hétérocyclique est présente dans la phase vapeur; et

(c) on élimine en continu du réacteur l'amine hétérocyclique produite.

2. Procédé de préparation de morpholine par contact d'un alcool qui est un diéthylèneglycol et/ou du 2-(2-amino-éthoxy)éthanol avec de l'ammoniac dans un réacteur, en présence d'hydrogène et d'un catalyseur d'hydrogénation-déshydrogénation, caracterisé en ce que:

(a) on fait passer en continu l'alcool, l'ammoniac et l'hydrogène à travers un réacteur à lit à ruissellement;

(b) on fait fonctionner le réacteur de telle sorte que les conditions de réaction ne dépassent pas de plus de 10°C le point de rosée de l'alimentation et qu'au moins 1% de l'alcool soit maintenu sous forme de phase liquide discontinue et au moins une portion de la morpholine est présente dans la phase vapeur; et

(c) on élimine en continu la morpholine du réacteur.

3. Procédé selon la revendication 1, caractérisé en ce que l'on fait fonctionner le réacteur à une température de 140 à 280°C sous une pression de $8{,}6 \times 10^5$ à $34 \times 10^5$ Pa (125 à 500 psig).

4. Procédé selon la revendication 2, caractérisé en ce que l'on fait fonctionner le réacteur à une température de 140 à 280°C sous une pression de $8{,}6 \times 10^5$ à $34 \times 10^5$ Pa (125 à 500 psig).

5. Procédé selon l'une quelconque des revendications précédentes, caractérisé en ce que plus de 20% en moles d'amine hétérocyclique formée pendant la réaction se trouvent dans la phase vapeur.

6. Procédé selon l'une quelconque des revendications précédentes, caractérisé en ce que l'alcool est du diéthylèneglycol.

7. Procédé selon l'une quelconque des revendications 1, 2, 5 et 6, caractérisé en ce que la température maintenue dans la zone de réaction est de 140 à 280 °C.

8. Procédé selon la revendication 7, caractérisé en ce que la pression maintenue dans la zone de réaction est de $8{,}6 \times 10^5$ à $34 \times 10^5$ Pa (125 à 500 psig).

9. Procédé selon l'une quelconque des revendications précédentes, caractérisé en ce que la vitesse spatiale horaire liquide, par rapport à la charge d'alimentation d'alcool, est de 0,05 à 2,5.

10. Procédé selon l'une quelconque des revendications précédentes, caractérisé en ce que le catalyseur d'hydrogénation-déshydrogénation est mouillable par l'alcool et qu'il est porté sur un support choisi parmi l'alumine, la silice, et leurs mélanges.

11. Procédé selon la revendication 10, caractérisé en ce que le catalyseur d'hydrogénation-déshydrogénation porté sur le support contient un composant choisi parmi le nickel, le cobalt et le chrome.

12. Procédé selon la revendication 11, caractérisé en ce que le catalyseur d'hydrogénation-déshydrogénation est un catalyseur de nickel sur support d'alumine.

13. Procédé selon l'une quelconque des revendications précédentes, caractérisé en ce que la zone de réaction est maintenue à 200–250 °C et que la pression est de $14 \times 10^5$ à $21 \times 10^5$ Pa (200–300 psig).

14. Procédé selon l'une quelconque des revendications précédentes, caractérisé en ce que l'amine hétérocyclique est la morpholine et qu'au moins 90% du nombre total de moles de morpholine dans le produit se trouvent dans la phase vapeur.

15. Procédé selon l'une quelconque des revendications précédentes, caractérisé en ce que le rapport molaire d'ammoniac à alcool dans les réacteurs est de 4:1 à 16:1.

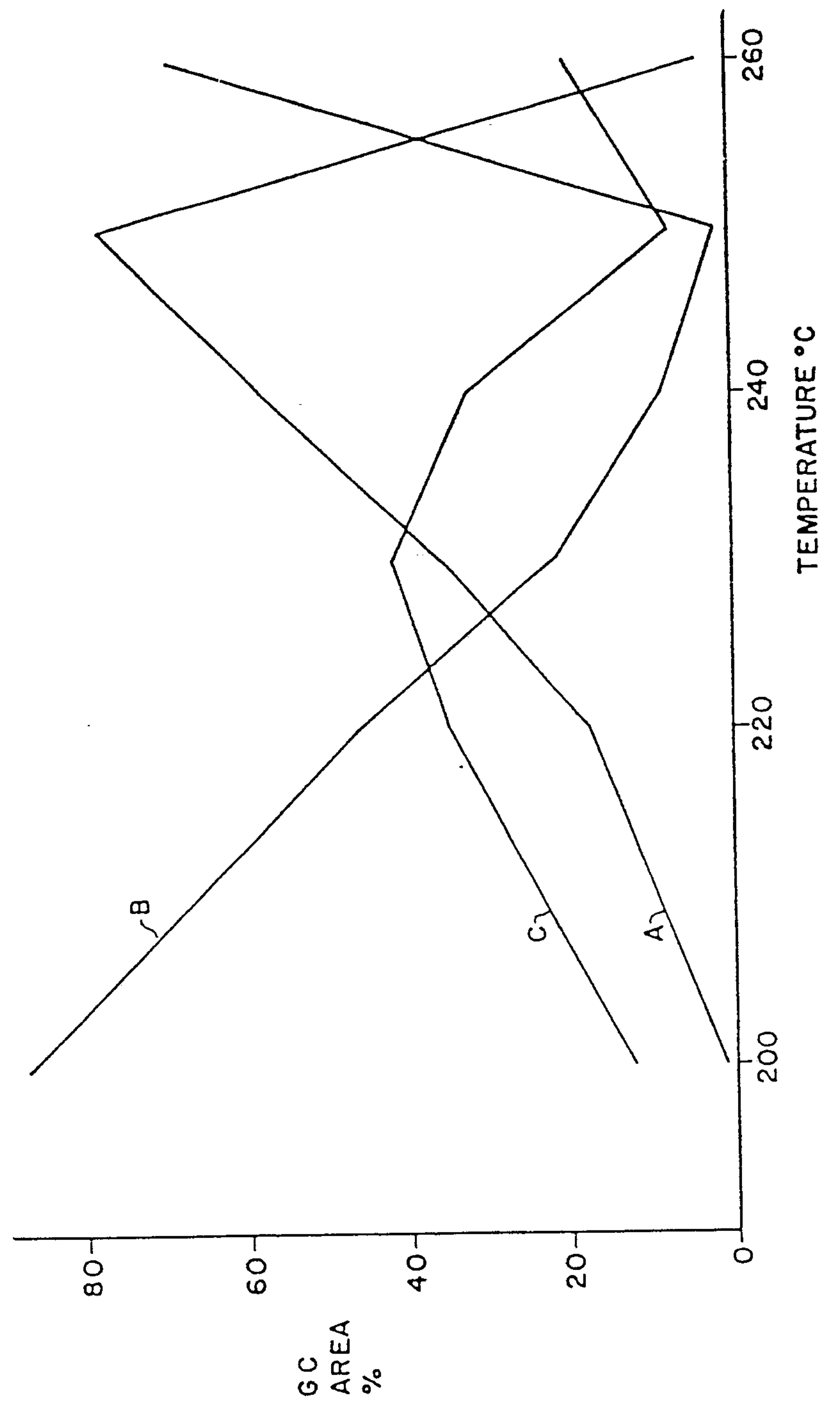


FIG. 1

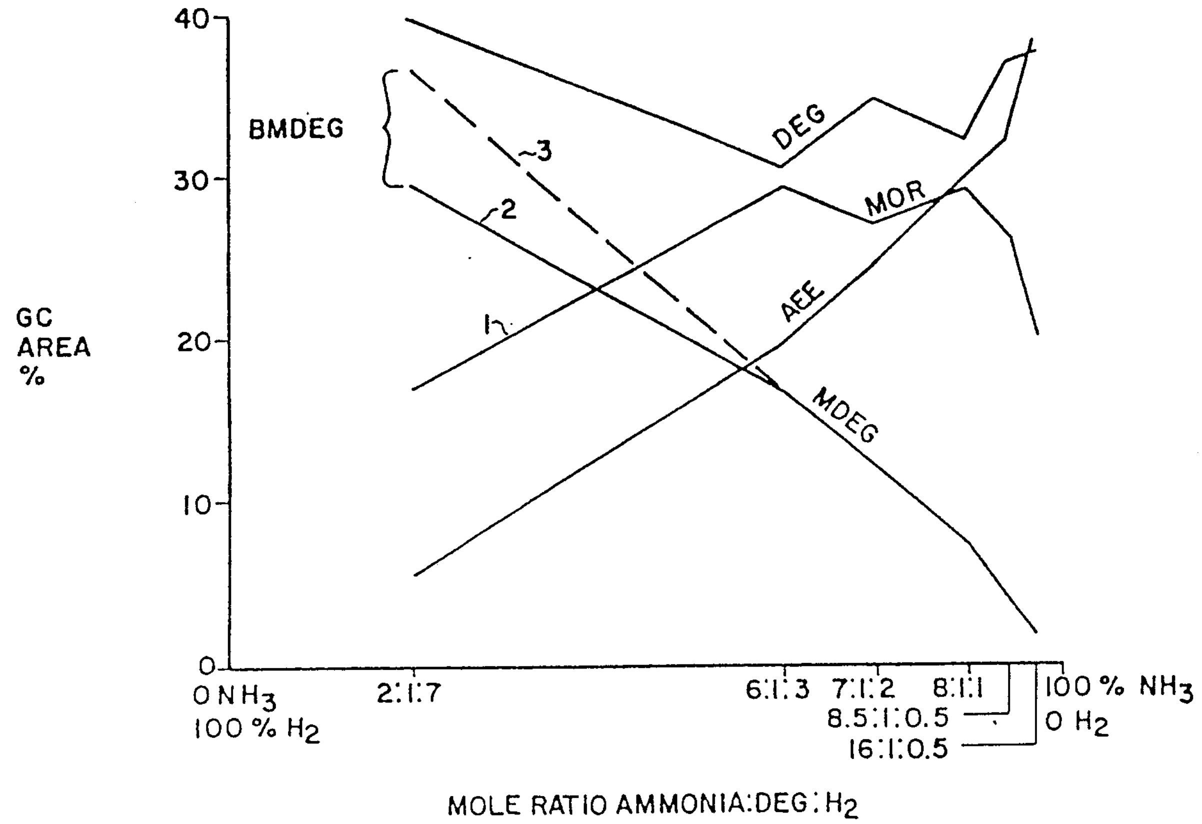
40
30
20
10
0
GC AREA %
BMDEG
~3
2
1
DEG
MOR
AEE
MDEG
0 NH3
100 % H2
2:1:7
6:1:3
7:1:2
8:1:1
8.5:1:0.5
16:1:0.5
100 % NH3
0 H2
MOLE RATIO AMMONIA:DEG:H2

FIG.2